Europäisches Patentamt

European Patent Office

Office européen des brevets

(19)

(11) Publication number: **0 303 488 B1**

(12) EUROPEAN PATENT SPECIFICATION

(45) Date of publication of patent specification: **28.12.94** (51) Int. Cl.⁵: **C07K 7/00, A61K 37/24**

(21) Application number: **88307447.8**

(22) Date of filing: **11.08.88**

(54) **Biologically active molecules.**

(30) Priority: **11.08.87 GB 8718937**

(43) Date of publication of application:
**15.02.89 Bulletin 89/07**

(45) Publication of the grant of the patent:
**28.12.94 Bulletin 94/52**

(84) Designated Contracting States:
**AT BE CH DE ES FR GB GR IT LI LU NL SE**

(56) References cited:
**WO-A-84/04915**

**JOURNAL OF ORGANIC CHEMISTRY, vol. 40, 1975; S.-S. WANG et al., pp. 1227-1234/**

**BIOCHEMISTRY, vol. 27, 1988, American Chemical Society; O.R.GOOLEY et al., pp. 802-809/**

**CHEMICAL ABSTRACTS, vol. 108, 1988, Columbus, OH (US); D.N. BREMA et al., p. 82, no. 180266w/**

**CHEMICAL ABSTRACTS, vol. 109, 1988, Columbus, OH (US); P.R. GOOLEY et al., p. 92, no. 222674f/**

(73) Proprietor: **MALLINCKRODT VETERINARY LIMITED**
**Breakspear Road South**
**Harefield**
**Uxbridge**
**Middlesex UB9 6LS (GB)**

(72) Inventor: **James, Stephen**
**Coopers Animal Health Ltd.,**
**Berkhamsted Hill**
**Berkhamsted,**
**Hertfordshire (GB)**
Inventor: **Bomford, Robert**
**The Wellcome Research Lab.,**
**Langley Court**
**Beckanham,**
**Kent (GB)**
Inventor: **Riley, John Hamilton**
**48 Green Avenue**
**Davenham**
**Nr. Northwich**
**Cheshire (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**CHEMICAL ABSTRACTS, vol. 106, 1987, Columbus, OH (US); R.W. STEVENSON et al., p. 99, no. 149835r/**

**CHEMICAL ABSTRACTS, vol. 100, 1984, Columbus, OH (US); W.P. VANDERLAAN et al., p. 71, no. 115141x/**

(74) Representative: **Matthews, Derek Peter et al**
**Frank B. Dehn & Co.**
**Imperial House**
**15-19 Kingsway**
**London WC2B 6UZ (GB)**

**Description**

The present invention relates to biologically active peptides.

Many polypeptide hormones are important medically or in animal husbandry, particularly growth hormones. Growth hormones are found in all vertebrates, the growth hormone (GH) of each species usually being slightly different. Generally speaking, the molecule comprises a single linear sequence of about 191 amino acids. The amino acid sequence of human growth hormone (hGH) is described by Choh Hao Li in "Molecular and Cellular Biochemistry", 46, 31-41 (1982). Growth hormones are known to enhance growth (somatogenesis), to enhance milk production (lactogenesis) and to have an insulin-like hypoglycemic effect.

It is known from EP A-137-234 that certain antibodies to growth hormone can potentiate the activity of the whole hormone. Such potentiating antibodies may be produced in situ by vaccinating the host animal with a suitable fragment of growth hormone, so that a class of polyclonal antibodies of restricted specificity is created, which will potentiate the activity of the endogenous hormone. A large (7K) fragment is disclosed in this earlier document as being suitable for such a purpose.

It is also known, for example from WO 84/04915 (EP-A-137 904), that peptides corresponding to portions of the GH molecule can have useful biological activity, in particular to generate hypoglycemic effects when administered contemporaneously with exogenous insulin. Such peptides are not administered in an antigenic formation, since the purpose is not to raise antibodies against the peptide.

It has now been found that particular portions of the GH molecule are especially suited to raising antibodies which will potentiate the activity of the hormone in a vertebrate. These peptides are substantially smaller than the 7K fragment referred to above, and hence can be synthesised more easily and cheaply. In addition, it has been found that the peptides generate antibodies which enhance the action of the hormone considerably more than do the antibodies to the 7K fragment referred to above.

The present invention provides a method for potentiating the effect of a pre-selected biologically active peptide, e.g. a hormone, in a vertebrate. The method uses small peptides containing predetermined sequences of amino acid residues that have primary structural homology with fragments of the biologically active peptide and comprises treating the vertebrate with a predetermined amount of the small peptide to enhance the effect of the pre-selected biologically active peptide.

The present invention also provides small peptides containing amino acid sequences of a hormone of a vertebrate, which small peptides potentiate the activity of the hormone in the vertebrate. Typically, the peptides of the invention are about 25 amino acid residues or less, and more preferably less than 20 amino acid residues. The number of amino acid residues that have structural homology with the hormone which are contained in the small peptides of the invention is typically dependent upon the length of the small peptide and may vary from a sequence of a few amino acid residues to a sequence substantially comprising the entire small peptide. Typically, the sequence of amino acid residues having the structural homology is at least 5 amino acid residues in length and preferably at least about 8 to 10 amino acid residues in length.

As used herein the term "potentiate" means that the small peptide acts directly or indirectly to increase or enhance the activity of the hormone to which it has the structural homology.

Accordingly, one aspect of the present invention provides a peptide corresponding to one of the following regions of bovine, ovine, porcine, human, avian or salmon growth hormone:

(a) 122-138
(b) 119-131
(c) 130-143
(d) 123-137
(e) 133-146

or an antigenically equivalent peptide wherein at least 45% of the peptide overlaps with at least 35% of any of the said peptides (a) to (e), or a variant of any of peptides (a) to (e) or of the said antigenically equivalent peptides having one or more conservative substitutions such that the tertiary configuration of the peptide is substantially unchanged.

The following are examples of the 122-138 region:

Bovine 122-138

```
ala-leu-met-arg-glu-leu-glu-asp-gly-thr-pro-arg-ala-gly-
gln-ile-leu
```

Ovine 122-138

This is the same as the bovine sequence except that it has 130-Val.

Porcine 122-138

```
ala-leu-met-arg-glu-leu-glu-asp-gly-ser-pro-arg-ala-gly-
gln-ile-leu
```

```
(N.B.  This sequence differs from bGH in having 131-ser)
```

Rat 122-138

```
ala-leu-met-gln-glu-leu-glu-asp-gly-ser-pro-arg-ile-gly-
gln-ile-leu
```

```
(N.B.  This sequence differs from pGH at 125-gln and
134-ile)
```

Avian 122-138

```
ala-leu-met-arg-glu-leu-glu-asp-arg-ser-pro-arg-gly-pro-
gln-leu-leu
```

The peptides according to the invention include analogues of growth hormone fragments which have conservative substitutions of one or more amino acids such that the tertiary configuration of the peptide is substantially unchanged. The term "conservative substitution" is defined functionally above. Examples of substitutions which may be conservative in this context include those having substantially the same hydrophobicity, size, charge and/or aromaticity as the original amino acid residue. All such substitutions and modifications are generally well known to those skilled in the art of peptide chemistry. For example, candidate substitutions include: proline for glycine and vice versa; alanine or valine for glycine and vice versa; isoleucine for leucine or methionine and vice versa (for example at position 124); tryptophan for tyrosine and vice versa; histidine for lycine and vice versa; serine for asparagine and vice versa; arginine for glutamate and vice versa; threonine for cysteine and vice versa; serine or alanine for threonine and vice versa (for example, Thr in place of Ala-134); and glutamine for asparagine and vice versa.

The peptides of the invention can be used, in a suitable formulation, to raise antibodies in a vertebrate, the antibodies acting to potentiate the growth-promoting or lactation-promoting action of growth hormone in that vertebrate. In particular, peptides which are slightly shorter or longer than the said regions or which

4

overlap substantially with the said regions, for example 119-131 or 130-143, have been found to be antigenically equivalent. The terms "slightly longer", "slightly shorter" and "substantial overlap" denote peptides in which at least 45% (preferably 50%, 60%, 70%, 80%, 90% or 100%) of the equivalent peptide overlaps with at least 35% (preferably 40%, 50%, 60%, 70%, 80%, 90% or 100%) of the said peptide regions. In particular, antigenically equivalent peptides which are shorter than the said fragments may be used.

It has been found that using a small peptide of the invention from a species other than the animal to which the peptide is being administered can be advantageous, for example, administering ovine peptides (e.g. 119-131 or 130-143 to pigs. Variations from the sequence of the animal's own GH may cause a greater immune response, whilst still yielding antibodies able to recognise the animal's own GH.

In addition, peptides in which one or more of the amino acid residues are chemically modified, before or after the peptide is synthesised, may be used providing that the function of the peptide, namely the production of specific antibodies in vivo, remains substantially unchanged. Such modifications include forming salts with acids or bases, especially physiologically acceptable organic or inorganic acids and bases, forming an ester or amide of a terminal carboxyl group, and attaching amino acid protecting groups such as N-t-butoxycarbonyl. Such modifications may protect the peptide from in vivo metabolism. The peptides may be present as single copies or as multiples, for example tandem repeats such as (122-138) + (122-138) or (122-138 + (138-122). Such tandem or multiple repeats may be sufficiently antigenic themselves to obviate the use of a carrier. It may be advantageous for the peptide to be formed as a loop, with the N-terminal and C-terminal ends joined together, or to add one or more Cys residues to an end to increase antigenicity and/or to allow disulphide bonds to be formed. If the peptide is covalently linked to a carrier, preferably a polypeptide, then the arrangement is preferably such that the peptide of the invention forms a loop.

A second aspect of the invention provides a pharmaceutical antigenic composition comprising one or more of any of the peptides of the first aspect of the invention, with means to provide carrier and adjuvant functions.

According to current immunological theories, a carrier function should be present in any immunogenic formulation in order to stimulate, or enhance stimulation of, the immune system. It is thought that carriers embody (or, together with the antigen, create) a T-cell epitope. The peptides may be associated, for example by cross-linking, with a separate carrier, such as serum albumins, myoglobins, bacterial toxoids and keyhole limpet haemocyanin. More recently developed carriers which induce T-cell help in the immune response include the hepatitis-B core antigen (also called the nucleocapsid protein), presumed T-cell epitopes such as Thr-Ala-Ser-Gly-Val-Ala-Glu-Thr-Thr-Asn-Cys, beta-galactosidase and the 163-171 peptide of interleukin-1. The latter compound may variously be regarded as a carrier or as an adjuvant or as both. Alternatively, several copies of the same or different peptides of the invention may be cross-linked to one another; in this situation there is no separate carrier as such, but a carrier function may be provided by such cross-linking. Suitable cross-linking agents include those listed as such in the Sigma and Pierce catalogues, for example glutaraldehyde, carbodiimide and succinimidyl 4-(N-maleimidomethyl)cyclohexane-1-carboxylate, the latter agent exploiting the -SH group on the C-terminal cysteine residue (if present).

If the peptide is prepared by expression of a suitable nucleotide sequence in a suitable host, then it may be advantageous to express the peptide as a fusion product with a peptide sequence which acts as a carrier. Kabigen's "Ecosec" system is an example of such an arrangement.

Suitable adjuvants are known to those in the vaccine art, for example Freund's complete or incomplete adjuvant, aluminium hydroxide, saponin, DEAE-dextran, muramyl dipeptide, mineral oils, neutral oils (such as miglyol), vegetable oils (such as arachis oil), "Iscoms", liposomes, Pluronic polyols or the Ribi adjuvant system (see, for example GB-A-2 189 141). "Pluronic" is a Registered Trade Mark.

The peptide of the invention maybe linked to other antigens to provide a dual effect. For example, the peptide may be linked to part or all of the somatostatin molecule to create, in addition to anti-GH antibodies, anti-somatostatin antibodies which would promote growth, or it may be linked to part or all of a sex hormone molecule to provide for simultaneous immunocastration, or to part or all of the luteinising hormone releasing hormone (LHRH).

The peptides and adjuvants and/or carriers may be formulated in any suitable way which may be devised by the man skilled in the art using known or yet-to-be-discovered delivery vehicles and criteria. In particular, the formulations may be based on biodegradable polymers such as lactide glycolide copolymers, such as those described in EP-A-58481 (ICI).

A further aspect of the invention provides a method of enhancing somatogenesis or lactogenesis, non-therapeutically, in a non-human vertebrate, comprising administering a pharmaceutical antigenic formulation according to the invention as described above. The peptides may be used to enhance the activity of endogenous and/or exogenously-administered GH, the latter being pituitary-extracted or rDNA-derived. The proportion lean mean to fat in an animal may also be enhanced by using such methods.

The small peptides and antigenic compositions of the invention will usually be administered intravenously, sub-cutaneously or intra-muscularly although intra-nasal, transdermal, oral and rectal routes may be suitable for some formulations of the invention. The formulation will normally be sterile and (for parenteral use) non-pyrogenic and a unit dose will typically include 1 to 1000 ug of the small peptide of the invention, typically 10 to 500 ug, preferably about 50 ug or less. One or more repeat immunisations may be advantageous, as is known in the art of immunology. The formulations may generally be prepared and/or administered by a physician or veterinary surgeon according to his skill and expertise.

A further aspect of the invention provides a process for preparing one of the peptides mentioned above, by known methods of peptide synthesis or by appropriate cleavage of the native GH molecule. Peptide synthesis may be achieved according to the general method of Stewart et al, described in "Solid Phase Peptide Synthesis" (W.H. Freeman, San Fransisco, 1969) or by the methods described by Marglin and Merrifield in Annual Reviews of Biochemistry 39, 841-866 at 862 (1970), and subsequent articles. Established methods of peptide synthesis by solid phase and similar techniques are usually not suitable for large scale production (although they may become so in the future) and thus commercial production of the peptides would normally be by cultivation of a suitable organism transformed with a polynucleotide sequence encoding the desired peptide. Thus, further aspects of the invention include such polynucleotides, transformation and expression vectors carrying such polynucleotides, organisms transformed therewith and processes for cultivating such organisms.

Examples in accordance with the invention will now be described, with reference to the accompanying drawings, in which:

Figure 1: Indication of antibody in 3 sheep (bovine 122-138 + cys immunised) over 270 day period. Counts per minute (cpm) are a measure of labelled GH bound to antibody precipitated and thereby an indication of antibody titre. These, and the control values, were normalised for an anti-GH sheep sera used throughout this series of experiments.

Figure 2: Hyphophysectomised rat growth experiment to show effect of diluting anti-bovine 122-138 Cys ovine anti sera on bovine growth hormone enhancement.

Bars represent 1 s.d.

Figure 3: Hypophysectomised rat growth experiment to show the enhancing effect of anti-porcine 122-138 Cys porcine anti-sera upon porcine GH induced growth using recombinant DNA derived or pituitary extracted hormone.

Bars represent 1 s.d.

Figure 4: Hypophysectomised rat growth experiment to show the pGH enhancing effect of porcine antisera (IgG globulin) from pigs immunised with 122-138 and related peptides. Note that recombinant pGH ( = 15 ug/rat/day) was used in this experiment.

Bars represent 1 s.d.

Figure 5: Hypophysectomised rat growth experiment to show the bGH enhancing effect of ovine anti-sera (IgG fraction) raised to a range of 122-138 and related peptides which recognise the bovine hormone. An exclusively porcine GH, recognising anti-sera raised in sheep, is also shown for comparison. This is a composite figure representing results from two experiments - for all treatments n = 6, for control globulin n = 12.

Figure 6: Hyphophysectomised rat growth experiment to show the consistent GH enhancing properties of pooled ovine anti-bovine 122-138 Cys antisera. Sera were pooled to give an equal total protein contribution from each animal and then the IgG fraction prepared as described in the text to give a final protein concentration in use of 10mg/ml. Bars represent 1 s.d., n = 6.

METHODS

Preparation of peptides

Unless otherwise indicated, all peptides were synthesised by the Fmoc-polyamide mode of solid-phase peptide synthesis.

Temporary N-amino group protection is afforded by the 9-fluorenylmethyloxycarbonyl (Fmoc) group. Repetitive cleavage of this highly base-labile protecting group is effected using 20% piperidine in N,N-dimethylformamide.

Side-chain functionalities are protected as their butyl ethers (in the case of serine, threonine and tyrosine), butyl esters (in the case of glutamic acid and aspartic acid), butyloxycarbonyl derivative (in the case of lysine and histidine), trityl derivative (in the case of cysteine) and 4-methoxy-2,3,6-trimethylben-zenesulphonyl derivative (in the case of arginine). Where glutamine or asparagine are C-terminal residues, use is made of the 4,4'-dimethoxybenzhydryl group for protection of the side chain amido functionalities.

The solid-phase support is based on a polydimethylacrylamide polymer constituted from the three monomers dimethylacrylamide (backbone-monomer), bis,acryloylethylene diamine (cross linker) and ac-ryloylsarcosine methyl ester (functionalising agent).

The peptide-to-resin cleavable linked agent used is the acid-labile 4-hydroxymethyl-phenoxyacetic acid derivative.

All amino acid derivatives are added as their preformed symmetrical anhydride derivatives with the exception of asparagine and glutamine that are added using a reversed N,N-dicyclohexylcarbodiimide/1-hydroxybenzotriazole mediated coupling procedure.

All coupling and deprotection reactions are monitored using ninhydrin, trinitrobenzene sulphonic acid or isotin test procedures.

Upon completion of synthesis, peptides are cleaved from the resin support with concomitant removal of side-chain protecting groups by treatment with 95% trifluoroacetic acid containing a 5% scavenger mix. Scavengers commonly used are ethanedithiol, phenol, anisole and water, the exact choice depending on the constituent amino acids of the peptide being synthesised. Trifluoroacetic acid is removed by evaporation in vacuo, with subsequent trituration with diethyl ether affording the crude peptide. Any scavengers present are removed by a simple extraction procedure which on lyophilisation of the aqueous phase affords the crude peptide free of scavengers.

Purification may be effected by any one, or a combination of, techniques such as size exclusion chromatography, ion-exchange chromatography and (principally) reverse-phase high performance liquid chromatography.

Analysis of peptides may be carried out using thin layer chromatography, reverse-phase hgh performance liquid chromatography, amino-acid analysis after acid hydrolysis and by fast atom bombardment (FAB) mass spectrometric analysis.

## I - SHEEP EXPERIMENTS : INVESTIGATORY

### Methods

#### 1. Conjugation to ovalbumin.

3.0mg of peptide was dissolved in 300ul of dimethyl formamide. 150ul of 10mg/ml ovalbumin in Dulbecco's phosphate buffered saline (PBS) was added and thoroughly mixed. 250ul of freshly prepared 0.04M glutaraldehyde was added slowly, with stirring, over a period of 10 minutes then left at room temperature for a further 60 minutes with with continuous mixing (SPIRAMIX, Denley Instruments). 1.0ml of PBS was added and followed by a further 100ul 0.04M glutaraldehyde as above. This was left for 60 minutes at room temperature before being dialysed overnight at +4 C against PBS.

#### 2. Negative Controls

Negative control parallels for the above were produced by using no peptide with ovalbumin nd treating with glutaraldehyde as described above.

#### 3. Adjuvants & Administration - 'Freunds'

After dialysis, the volumes of the above preparations were made up to 4.5ml with PBS and 'water-in-oil' emulsions prepared using two volumes of Freund's Complete Adjuvant (FCA) (Difco or Sigma). This was achieved by sonication in the cold or using a Potter-Elvehjen homogeniser. Emulsions were tested by dispersion (or absence) on a water surface. The injections were subcutaneously administered at two sites (one on each flank) into Cheviot sheep (9-12 months old, castrate males, 30-35kg). 1ml was administered at each site.

Twenty eight (experiment 1) or thirty five (experiment 2) days after the primary immunisation a second, similar immunisation was completed using freshly prepared antigen conjugated in the same way but emulsified into Freund's Incomplete Adjuvant (FIA, Difco or Sigma). Subsequent boosts (experiment 2) were given at further intervals of twenty one and fifty three days, but were one-half and one-quarter of the original dose.

### 4. Blood Samples

10ml blood samples were taken by jugular venepuncture, from the sheep under test, just prior to any administration and at intervals thereafter (see results). After allowing the clot to form at room temperature (approximately 5 hours) the serum was removed after centrifugation for immediate antibody-detecting radio-immunoassay. Larger samples of sera were collected in the same way from approximately 150-200mls of blood, were frozen at-20 C for subsequent fractionation of IgG fraction for use in growth assays.

### 5. Radioimmunoassay

The detection of antibodies to peptides which would also bind to bovine growth hormone was determined by liquid phase direct binding as described previously (Aston et al, 1985; Chard, 1987).

### Results

Table 1 summarizes the result from the sheep experiments and indicates an antibody response in sheep to a wide range of peptides related to the 122-138 GH peptide. The ovine and bovine peptides at 122-138 produce a similar response but the additional, adjacent modification in the porcine GH 122-138 sequence produces fewer animals with antibodies to bovine GH. Interestingly, with porcine GH radioimmune assay this response was raised to 80%, suggesting that the 2 adjacent modifications (ovine to porcine) result in recognition problems not seen with the single ovine/bovine difference. The potential of a terminal Cysteine ("cys") in assisting the immune response is also illustrated in the 122-138 peptides as well as the highly conserved (and therefore potentially poorly antigenic) 133-146. Cross-species differences can partially overcome the lack of cysteine used in the 130-143 peptide without the damage to recognition observed when these are in the middle of the sequence.

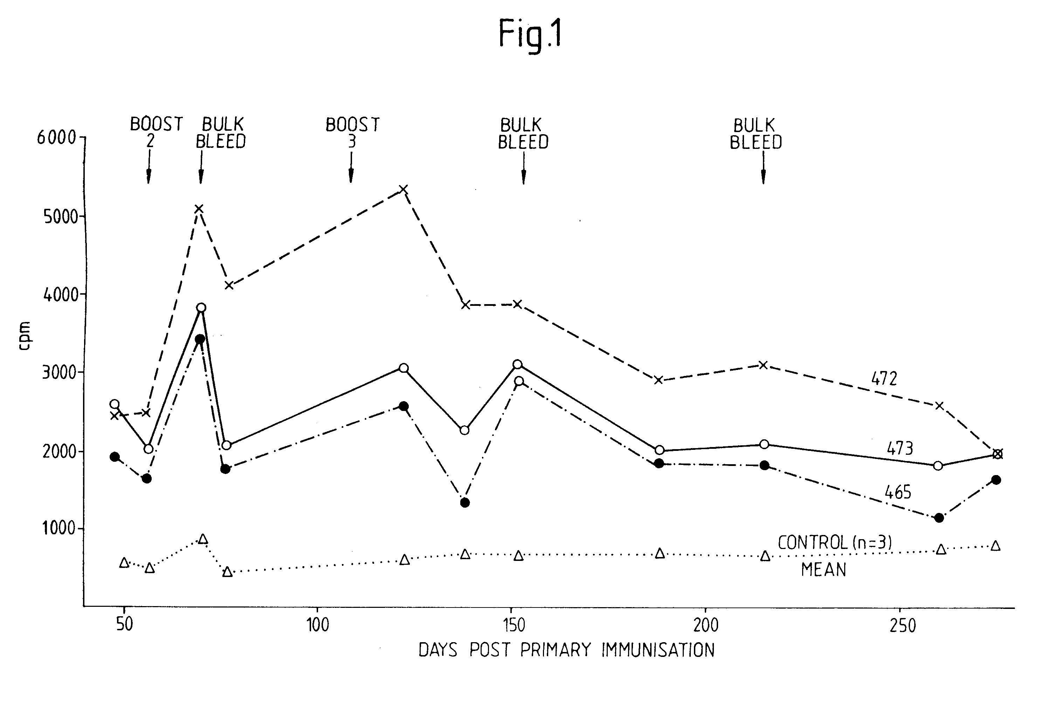

Figure 1 shows how the antibody response may be sustained for many months (experiment 2) by an appropriate number of boosts. In this experiment the presence of particular elevated antibody levels after the boosts is indicated in this figure. Pooled serum samples were used to demonstrate that GH-enhancing properties (see also hypophysectomised rat experiments) of the sera are retained for at least seven months after the primary immunisation.

Table 1

| Radioimmunoassay to detect antibodies in sheep sera which are capable of binding to intact bovine growth hormone in liquid phase. | | |
|---|---|---|
| Peptide treatment | | % Responders at 49 days [with titre of 1/500 or better ] n = 5. |
| Ovine 122-138 + Cys | | 80 |
| Bovine 122-138 + Cys | fmoc synthesis* <br> fboc synthesis | 80 <br> 80 |
| Porcine 122-138 + Cys <br> Bovine 122-138 + Cys <br> Ovine 130-143 + NONE <br> Bovine 130-143 + NONE <br> Porcine 130-143 + NONE <br> Ovine/bovine/porcine 133 = 146 = Cys | | 40 <br> 20 <br> 40 <br> 60 <br> 60 <br> 60 |

*All peptides were made by fmoc synthesis unless otherwise indicated.

## II PIG EXPERIMENT

### Methods

#### 1. Conjugation to Keyhole Limpet Haemocyanin (KLH)

A range of peptides were conjugated in the same way as previously described for the sheep, except that KLH was used as the carrier instead of ovalbumin. A 10mg/ml solution of KLH was prepared in PBS by rolling overnight with borosilicate glass beads (5mm diameter). Negative control treatments were prepared similarly except that the peptide was omitted.

#### 2. Adjuvants and Administration

After dialysis the volumes of the peptide + conjugate preparations were made up to 4.5ml with PBS and water-in-oil emulsions prepared using two volumes of Freund's Complete Adjuvant (Sigma). This was achieved by sonication after cooling the well shaken oil + aqueous peptide mix to 0 C and pre-cooling the probe of the sonicator. Two short (5 seconds) bursts at full power (SONIPREP 150, Gallenkamp, Loughborough, England) produced a stable emulsion ready to use in the pigs.

The peptide so prepared was administered subcutaneously at 4 sites in the neck region of large white piglets (5 weeks of age; approximately 9kg body weight) so as to give 500ug peptide per pig. A second immunisation using a similar, fresh, preparation was given 28 days later. On this second occasion all were delivered in FIA.

#### Blood Samples

Blood samples (10ml) were collected just prior to the second immunisation and weekly thereafter, by vacuum-assisted venepuncture (Corvac, Sarstedt, U.K.) of the ascending vena cava. The sera were tested for antibody recognition of porcine growth hormone using an Enzyme Linked Immunosorbent Assay (ELISA) based on Voller, 1979, which was subsequently cross-checked by competition, in a similar assay, with aqueous hormone.

#### 4. ELISA

96-well plates treated for immunoassay consistency (Nunc, Immuno-quality, High-binding capacity) were coated using 50ug hormone/ml at 5ug.well (100ml) in sodium carbonate/bicarbonate buffer 0.05M pH9.5 and allowed to stand overnight at +4 C. The hormone solution was carefully removed and the wells washed once with PBS. A solution of 3% haemoglobin was added to 'block' the wells and left overnight at +4 C. This was removed and the wells washed three times with PBS plus 0.05% Tween® 20. All plates were allowed to dry slowly at room temperature and stored at -20 C in a dessicator cabinet individually wrapped in cling-film. Sera under test were added to each of the wells at 1/5th and subsequent log dilutions (100ul) and left for 2 hours, at room temperature. This was removed and the wells washed three times in PBS, and replaced by 100ul rabbit anti-pig IgG alkaline phosphate conjugate (Sigma) at 10 dilution. This was removed after minutes and washed as before. 100ul of p-nitrophenyl phosphate at 1.0mg/ml was added and the absorbance of the wells read using Titertek Multiscan Plus 2 with 405nm filter.

#### Results

Table 2 shows that the presence of antibodies which recognised coated porcine growth hormone could be detected in a number of pigs. Sera were considered positive when absorbances were more than twice that of controls and this could be reversed by the prior addition in the system of aqueous porcine growth hormone.

It can be seen that the C-terminal cepteine of the sequence 122-138 is very significant in improving the production of antibodies which recognise porcine GH. Various other modifications can also be employed to the same effect and these include cross-species changes towards either the C-terminal (eg comparison of porcine and ovine 119-131) or the N-terminal (eg as in 130-143). Similarly artificial modifications may be introduced which can improve antigenicity without necessarily loss of entire hormone recognition. This can be seen by comparison of responses achieved using the p122-138 (only) and p123-137+Thr, in which methionine-124 has been replaced with an isoleucine during peptide synthesis.

Table 3

Anti porcine GH antibodies in sera from peptide immunised pigs at 49 days past primary immunisation as measured by the ELISA.

Peptide treatment
(all fmoc synthesis unless indicated)

% Positive Animals
(n=6, unless indicated)

p = porcine
b = bovine

| Peptide treatment | 1/50 * | 1/500 * |
|---|---|---|
| p122-138 + cys (fboc synthesis) | 83 | 83 |
| p122-138 + cys (n=5) | 80 | 80 |
| p122-138 (only) | 66 | 0 |
| p119-131 + cys | 100 | 66 |
| b119-131 + cys | 100 | 100 |
| p130-143 (only) | 50 | 17 |
| b130-143 | 66 | 33 |
| p123-137 + thr (met 124 = Ile) | 83 | 50 |

*antisera dilution

III - BIOLOGICAL ASSAY OF GH ACTIVITY

Methods

1. Immunoglobulin Preparations

Sera from larger blood samples taken from particular animals (indicated by the immunoassays) were fractionated by sodium sulphate precipitation (Johnstone & Thorpe, 1982) to isolate principally the gamma-globulins (IgG) which were extensively dialysed against PBS before being frozen at -20oC. Prior to use in animal experiments the purified IgG fractions were re-titrated to monitor the effects, if any, of precipitation and storage.

2. Hypophysectomised Rat

These animals are rendered pituitary (hypophysis) deficient by surgical removal. The assay monitors the overall effect of the hormone on body weight of the rat.

The surgery on male, Wistar rats was completed by Charles River U.K. Limited (Margate, Kent, U.K.) and delivered 14 days later at a weight range of approximately 110-145g. They were weighed and observed for a further 7-14 days to ensure stable body weight and physical features (for example, non-appearance of testicles) consistent with good health and complete surgery. Satisfactory animals were randomly allocated to provide six animals per treatment. Overall weight ranges 20g live weight, or less, were used in individual experiments. These were injected daily with 0.5ml PBS containing IgG from the immunisation treatment under study (including negative controls), to which had been added 50ug (exceptionally 15ug if recombinant

10

material used - see results) bovine or porcine growth hormone and IgG solutions were mixed thoroughly together and allowed to stand at room temperature for 60 minutes. Injections were subcutaneous and intrascapular. Animals were weighed and injected daily for 9 days, at the same time of day on each occasion.

Results

Using this growth-monitoring bio-assay it can be seen that IgG from a variety of 122-138 and related anti-peptide sera which also recognise the appropriate intact growth hormone molecule will enhance the somotogenic activity of this hormone. This sort of bioassay has been widely used as a measure of the biological activity of growth hormones from various species (M.D. Groesbeck and A.F. Parlow, 1987, Endocrinology 120 2582-2590).

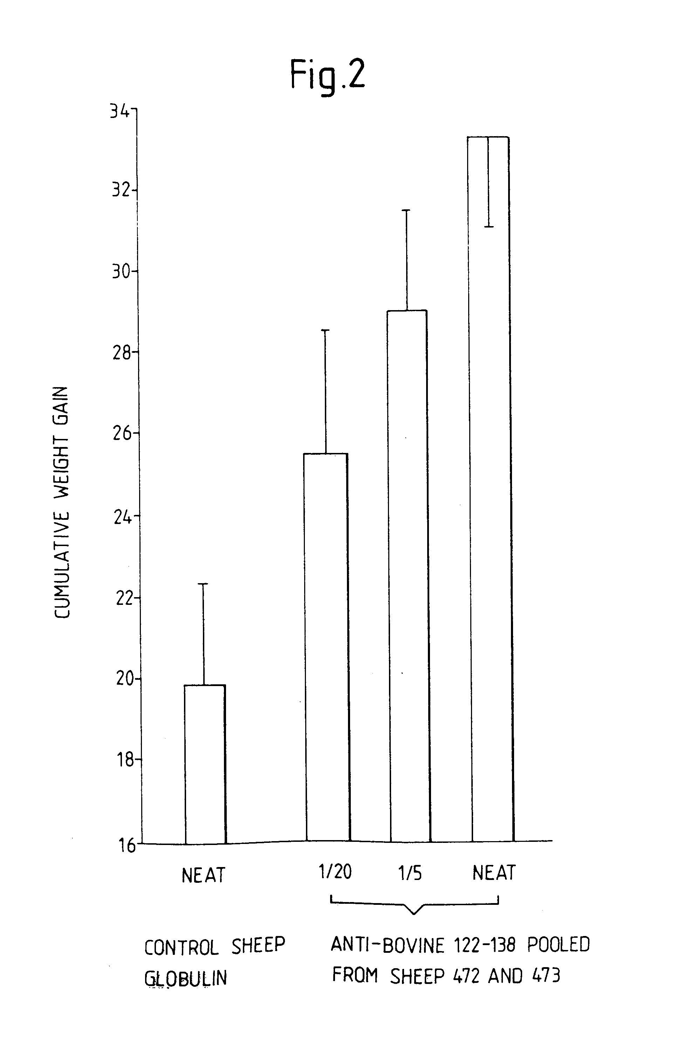

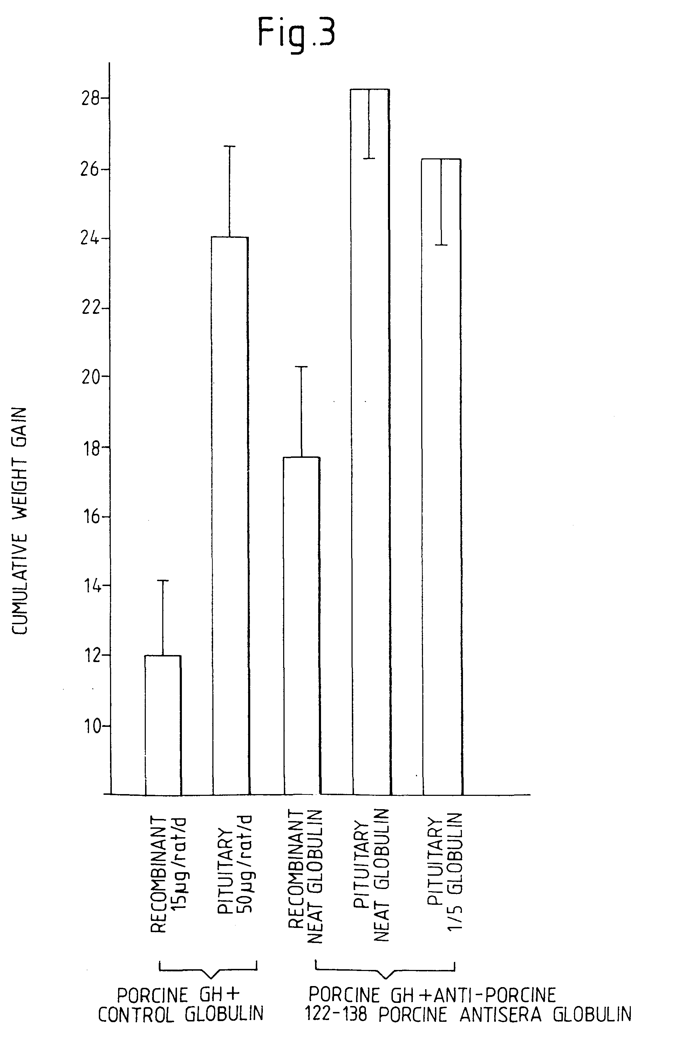

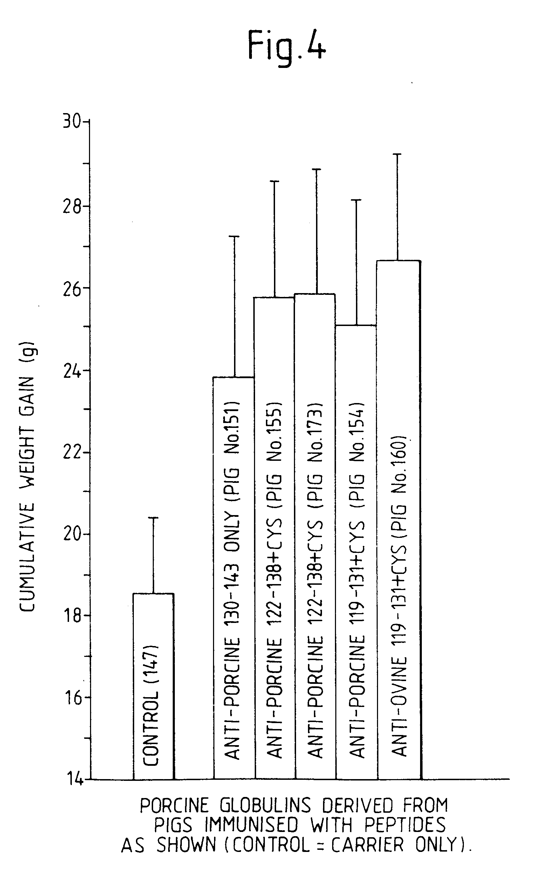

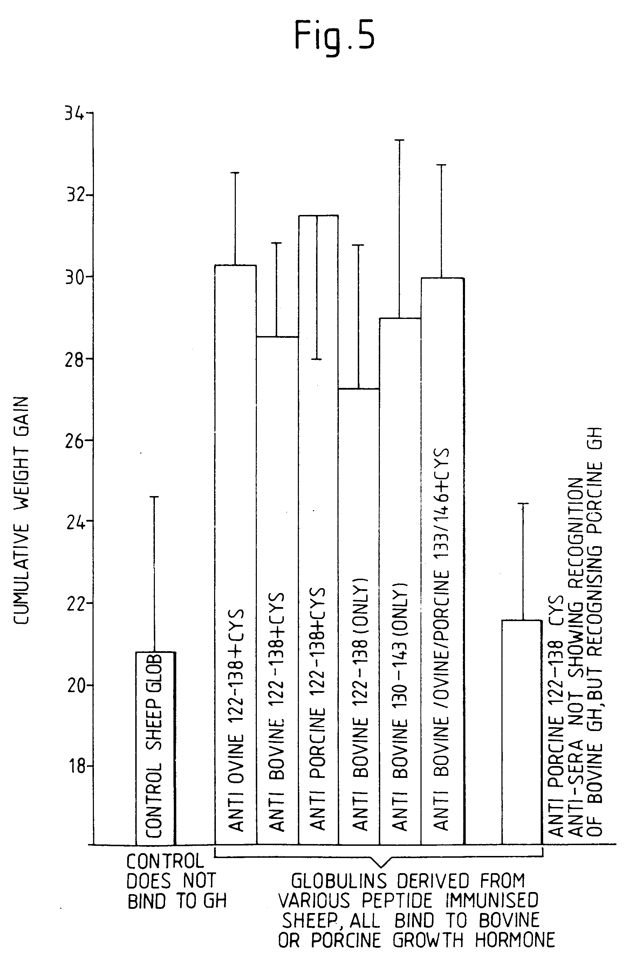

The effect is sensitive to dilution of the globulin fraction (Figure 2) and is applicable to both porcine and bovine growth hormones (Figures 2-6) as well as growth hormone derived from recombinant DNA sources (Figures 3 and 4). That this phenomenon will cross species barriers, provided that the antisera will bind to the target hormone in question, is well demonstrated in Figure 5: Antisera raised in a sheep after immunising with porcine 122-138 + cys failed to show an ability to bind bovine growth hormone (but would bind to the porcine version); this IgG fraction failed to show an enhancing ability when administered with bovine GH, but antisera from another sheep raised to the same peptide and capable of recognising bovine GH did show an enhancing activity towards this hormone.

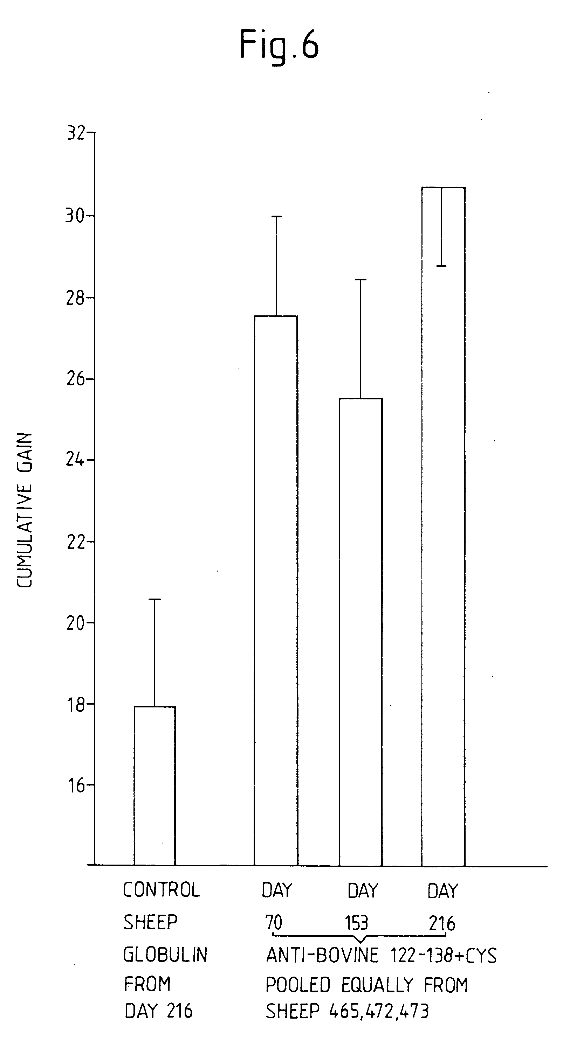

Figure 6 illustrates results from a hypophysectomised rat experiment in which pooled antisera from three sheep showing antibodies to bovine GH after administration of bovine 122-138 were used. It can be seen how bovine GH-enhancing properties are retained for over 200 days without any indication of reduction.

References

1. Aston, R., Cooper, L., Holder, A.T., Ivanyi, J., and Preece, M.A. (1985). Molecular Immunol. 22 271-275.
2. Aston, R., Holder, A.T., Preece, M.A., and Ivanyi, J. (1986) J. Endocrinol. 110 381-388.
3. Aston., R., Holder, A.T., Ivanyi, J. and Bomford, R (1987). Molec. Immunol. 24 143-150.
4. Chard, T (1987). An Introduction to Radio-Immunoassay and Related Techniques. 3rd Edition. Elsevier, Amsterdam.
5. Johnstone, A., and Thorpe, R. (1982). Immunochemistry in Practice, Blackwells, London.
6. Voller, A., Bidwell, D.E. and Bartlett, A. (1979). The Enzyme Linked Immunosorbent Assay, Dynatech Europe, Guernsey.

**Claims**
**Claims for the following Contracting States : AT, BE, CH, DE, FR, GB, IT, LI, LU, NL, SE**

**1.** A peptide corresponding to one of the following regions of bovine, ovine, porcine, human, avian or salmon growth hormone:
    (a) 122-138
    (b) 119-131
    (c) 130-143
    (d) 123-137
    (e) 133-146
or an antigenically equivalent peptide wherein at least 45% of the peptide overlaps with at least 35% of any of the said peptides (a) to (e), or a variant of any of peptides (a) to (e) or of the said antigenically equivalent peptides having one or more conservative substitutions such that the tertiary configuration of the peptide is substantially unchanged.

**2.** A peptide according to Claim 1 having from 5 to 20 amino acid residues.

**3.** A peptide according to Claim 1 or 2 and additionally comprising a terminal cysteine residue.

**4.** A peptide according to any one of the preceding claims conjugated to (a) itself, (b) another peptide according to any one of the preceding claims, (c) a T-cell epitope or (d) part or all of the somatostatin

EP 0 303 488 B1

molecule.

5. A pharmaceutical antigenic formulation comprising a means to provide adjuvant and carrier functions and a peptide according to any one of the preceding claims.

6. A formulation according to Claim 5 wherein the peptide is linked to a carrier.

7. A formulation according to Claim 5 or 6 wherein the peptide, whether or not linked to a carrier, is mixed with an adjuvant.

8. A non-therapeutic method of enhancing somatogenesis or lactogenesis in a non-human vertebrate comprising administering to the vertebrate a formulation according to any of the Claims 5 to 7.

9. A process for preparing a peptide according to any one of Claims 1 to 4 by chemical peptide synthesis or by cultivating a suitably transformed organism.

10. A polynucleotide sequence encoding a peptide according to any one of Claims 1 to 4.

**Claims for the following Contracting States : ES, GR**

1. A process for preparing a peptide corresponding to one of the following regions of bovine, ovine, porcine, human, avian or salmon growth hormone:
   (a) 122-138
   (b) 119-131
   (c) 130-143
   (d) 123-137
   (e) 133-146
   or an antigenically equivalent peptide wherein at least 45% of the peptide overlaps with at least 35% of any of the said peptides (a) to (e), or a variant of any of peptides (a) to (e) or of the said antigenically equivalent peptides having one or more conservative substitutions such that the tertiary configuration of the peptide is substantially unchanged
   by (i) proteolytic degradation of a larger peptide, (ii) chemical synthesis from amino acids or (iii) expression of a nucleotide sequence coding for the said peptide in a suitable host.

2. A process according to Claim 1 wherein the peptide has between 5 and 20 amino acid residues.

3. A process according to Claim 1 or Claim 2 wherein the peptide additionally comprises a terminal cysteine residue.

4. A process according to any one of the preceding claims wherein the peptide is conjugated to (a) itself, (b) another peptide prepared according to any one of the preceding claims, (c) a T-cell epitope or (d) part or all of the somatostatin molecule.

5. A process for preparing a pharmaceutical antigenic formulation comprising bringing together a peptide prepared according to any one of the preceding claims and means to provide adjuvant and carrier functions.

6. A process according to Claim 5 wherein the peptide is linked to a carrier.

7. A process according to Claim 5 or 6 wherein the peptide, whether or not linked to a carrier, is mixed with an adjuvant.

8. A non-therapeutic method of enhancing somatogenesis or lactogenesis in a non-human vertebrate comprising administering to the vertebrate a formulation according to any one of Claims 5 to 7.

12

**Patentansprüche**
**Patent Ansprüche für folgende Vertragsstaaten : AT, BE, CH, DE, FR, GB, IT, LI, LU, NL, SE**

1. Peptid, das einer der folgenden Regionen des Wachstumshormons vom Rind, Schaf, Schwein, Mensch, Vogel oder Lachs entspricht
   (a) 122-138
   (b) 119-131
   (c) 130-143
   (d) 123-137
   (e) 133-146
   oder antigen äquivalentes Peptid, worin mindestens 45 % des Peptids mit mindestens 35 % eines dieser Peptide (a) bis (e) überlappen, oder eine Variante eines dieser Peptide (a) bis (e) oder dieser antigen äquivalenten Peptide, die eine oder mehrere konservative Substitutionen so aufweist, daß die tertiäre Konfiguration des Peptids im wesentlichen unverändert ist.

2. Peptid nach Anspruch 1, das 5 bis 20 Aminosäurereste aufweist.

3. Peptid nach Anspruch 1 oder 2, das zusätzlich einen terminalen Cysteinrest umfaßt.

4. Peptid nach einem der vorangehenden Ansprüche, das mit (a) sich selbst, (b) einem anderen Peptid nach einem der vorangehenden Ansprüche, (c) einem T-Zellepitop oder (d) einem Teil oder dem gesamten Somatostatinmolekül konjugiert ist.

5. Antigene pharmazeutische Formulierung, die ein Mittel zur Bereitstellung von Adjuvans- und Trägerfunktionen und ein Peptid nach einem der vorangehenden Ansprüche umfaßt.

6. Formulierung nach Anspruch 5, worin das Peptid an einen Träger gebunden ist.

7. Formulierung nach Anspruch 5 oder 6, worin das Peptid, ob an den Träger gebunden oder nicht, mit einem Adjuvans gemischt ist.

8. Nicht-therapeutisches Verfahren zur Steigerung der Somatogenese oder Lactogenese in einem nichthumanen Vertebraten, das die Verabreichung einer Formulierung nach einem der Ansprüche 5 bis 7 an den Vertebraten umfaßt.

9. Verfahren zur Herstellung eines Peptids nach einem der Ansprüche 1 bis 4 durch chemische Peptidsynthese oder durch Kultivierung eines geeignet transformierten Organismus.

10. Polynukleotidsequenz, die ein Peptid nach einem der Ansprüche 1 bis 4 kodiert.

**Patentansprüche für folgende Vertragsstaaten : ES, GR**

1. Verfahren zur Herstellung eines Peptids, das einer der folgenden Regionen des Wachstumshormons vom Rind, Schaf, Schwein, Mensch, Vogel oder Lachs entspricht
   (a) 122-138
   (b) 119-131
   (c) 130-143
   (d) 123-137
   (e) 133-146
   oder eines antigen äquivalenten Peptids, worin mindestens 45 % des Peptids mit mindestens 35 % eines dieser Peptide (a) bis (e) überlappen, oder einer Variante eines dieser Peptide (a) bis (e) oder dieser antigen äquivalenten Peptide, die eine oder mehrere konservative Substitutionen so aufweist, daß die tertiäre Konfiguration des Peptids im wesentlichen unverändert ist, durch
   (i) proteolytischen Abbau eines größeren Peptids, (ii) chemische Synthese aus Aminosäuren oder (iii) Expression einer Nukleotidsequenz in einem geeigneten Wirt, die für dieses Peptid kodiert.

2. Verfahren nach Anspruch 1, worin das Peptid 5 bis 20 Aminosäurereste aufweist.

**3.** Verfahren nach Anspruch 1 oder 2, worin das Peptid zusätzlich einen terminalen Cysteinrest umfaßt.

**4.** Verfahren nach einem der vorangehenden Ansprüche, worin das Peptid mit (a) sich selbst, (b) einem anderen nach einem der vorangehenden Ansprüche hergestellten Peptid, (c) einem T-Zellepitop oder (d) einem Teil oder dem gesamten Somatostatinmolekül konjugiert ist.

**5.** Verfahren zur Herstellung einer antigenen pharmazeutischen Formulierung, das das Zusammenbringen eines nach einem der vorangehenden Ansprüche hergestellten Peptids mit einem Mittel zur Bereitstellung von Adjuvans- und Trägerfunktionen umfaßt.

**6.** Verfahren nach Anspruch 5, worin das Peptid an einen Träger gebunden ist.

**7.** Verfahren nach Anspruch 5 oder 6, worin das Peptid, ob an den Träger gebunden oder nicht, mit einem Adjuvans gemischt wird.

**8.** Nicht-therapeutisches Verfahren zur Steigerung der Somatogenese oder Lactogenese in einem nichthumanen Vertebraten, das die Verabreichung einer Formulierung nach einem der Ansprüche 5 bis 7 an den Vertebraten umfaßt.

**Revendications**
**Revendications pour les Etats contractants suivants : AT, BE, CH, DE, FR, GB, IT, LI, LU, NL, SE**

**1.** Peptide correspondant à l'une des régions suivantes de l'hormone de croissance bovine, ovine, porcine, humaine, aviaire ou de saumon :
   (a) 122-138
   (b) 119-131
   (c) 130-143
   (d) 123-137
   (e) 133-146
   ou d'un peptide équivalent du point de vue antigénique, dans lequel au moins 45 % du peptide recouvre au moins 35 % de l'un quelconque desdits peptides (a) à (e), ou un variant de l'un quelconque des peptides (a) à (e) ou desdits peptides équivalents du point de vue antigénique ayant une ou plusieurs substitutions conservatrices telle(s) que la configuration tertiaire du peptide est essentiellement inchangée.

**2.** Peptide selon la revendication 1 comportant 5 à 20 résidus d'acides aminés.

**3.** Peptide selon la revendication 1 ou 2, comprenant en outre un résidu cystéine terminal.

**4.** Peptide selon l'une quelconque des revendications précédentes, conjugué à (a) lui-même, (b) un autre peptide selon l'une quelconque des revendications précédentes, (c) un épitope de cellule T ou (d) tout ou partie de la molécule de somatostatine.

**5.** Composition pharmaceutique antigénique comprenant un moyen procurant les fonctions d'adjuvant et de support et un peptide selon l'une quelconque des revendications précédentes.

**6.** Composition selon la revendication 5, dans laquelle le peptide est lié à un support.

**7.** Composition selon la revendication 5 ou 6, dans laquelle le peptide, lié ou non à un support, est mélangé avec un adjuvant.

**8.** Méthode non-thérapeutique pour augmenter la somatogénèse ou la lactogénèse chez un vertébré non-humain qui comprend l'administration au vertébré d'une composition selon l'une quelconque des revendications 5 à 7.

**9.** Procédé de préparation d'un peptide selon l'une quelconque des revendications 1 à 4 par synthèse peptidique chimique ou par culture d'un organisme transformé de façon appropriée.

**10.** Séquence polynucléotidique codant pour un peptide selon l'une quelconque des revendications 1 à 4.

**Revendications pour les Etats contractants suivants : ES, GR**

**1.** Procédé de préparation d'un peptide correspondant à l'une des régions suivantes de l'hormone de croissance bovine, ovine, porcine, humaine, aviaire ou de saumon :

(a) 122-138
(b) 119-131
(c) 130-143
(d) 123-137
(e) 133-146

ou d'un peptide équivalent du point de vue antigénique, dans lequel au moins 45 % du peptide recouvre au moins 35 % de l'un quelconque desdits peptides (a) à (e), ou un variant de l'un quelconque des peptides (a) à (e) ou desdits peptides équivalents du point de vue antigénique ayant une ou plusieurs substitutions conservatrices telle(s) que la configuration tertiaire du peptide est essentiellement inchangée,

par (i) dégradation protéolytique d'un peptide plus grand, (ii) synthèse chimique à partir d'aminoacides ou (iii) expression d'une séquence nucléotidique codant pour ledit peptide dans un hôte approprié.

**2.** Procédé selon la revendication 1, dans lequel le peptide comporte entre 5 et 20 résidus d'acides aminés.

**3.** Procédé selon la revendication 1 ou 2, dans lequel le peptide comprend en outre un résidu cystéine terminal.

**4.** Procédé selon l'une quelconque des revendications précédentes, dans lequel le peptide est conjugué à (a) lui-même, (b) un autre peptide préparé selon l'une quelconque des revendications précédentes, (c) un épitope de cellule T ou (d) tout ou partie de la molécule de somatostatine.

**5.** Procédé de préparation d'une composition pharmaceutique antigénique comprenant la réunion d'un peptide préparé selon l'une quelconque des revendications précédentes et d'un moyen procurant les fonctions d'adjuvant et de support.

**6.** Procédé selon la revendication 5, dans lequel le peptide est lié à un support.

**7.** Procédé selon la revendication 5 ou 6, dans lequel le peptide, lié ou non à un support, est mélangé avec un adjuvant.

**8.** Méthode non-thérapeutique pour augmenter la somatogénèse ou la lactogénèse chez un vertébré non-humain qui comprend l'administration au vertébré d'une composition selon l'une quelconque des revendications 5 à 7.

Fig.1

EP 0 303 488 B1

# Fig.2

# Fig.3

CUMULATIVE WEIGHT GAIN

RECOMBINANT 15μg/rat/d

PITUITARY 50μg/rat/d

RECOMBINANT NEAT GLOBULIN

PITUITARY NEAT GLOBULIN

PITUITARY 1/5 GLOBULIN

PORCINE GH +
CONTROL GLOBULIN

PORCINE GH +ANTI-PORCINE
122-138 PORCINE ANTISERA GLOBULIN

# Fig.4

CUMULATIVE WEIGHT GAIN (g)

CONTROL (147)

ANTI-PORCINE 130-143 ONLY (PIG No.151)

ANTI-PORCINE 122-138+CYS (PIG No.155)

ANTI-PORCINE 122-138+CYS (PIG No.173)

ANTI-PORCINE 119-131+CYS (PIG No.154)

ANTI-OVINE 119-131+CYS (PIG No.160)

PORCINE GLOBULINS DERIVED FROM
PIGS IMMUNISED WITH PEPTIDES
AS SHOWN (CONTROL = CARRIER ONLY).

# Fig.5

# Fig.6